# EUROPÄISCHE PATENTSCHRIFT

(11) **EP 0 560 109 B1**
(45) Veröffentlichungstag und Bekanntmachung des Hinweises auf die Patenterteilung: **17.07.1996**
(21) Anmeldenummer: 93102759.3
(22) Anmeldetag: 22.02.1993
(51) Int. Cl.: C07C 49/327, C07D 249/08

(54) **Verfahren zur Herstellung von 1-Fluorcyclopropyl-methyl-keton**
Process for the preparation of 1-fluorocyclopropyl-methyl-ketone
Procédé pour la préparation de 1-fluorocyclopropyl-méthyl-cétone

(30) Priorität: 05.03.1992 DE 4206917
(43) Veröffentlichungstag der Anmeldung: 15.09.1993
(60) Teilanmeldung: 96100322.5
(73) Patentinhaber: BAYER AG, 51368 Leverkusen (DE)
(72) Erfinder: Böhm, Stefan, Dr., W-5000 Köln 80 (DE); Marhold, Albrecht, Dr., W-5090 Leverkusen 1 (DE)

(56) Entgegenhaltungen:
- SYNTHESIS Nr. 3, 1977, Seiten 189 - 191 L. FITJER
- TETRAHEDRON LETT. Bd. 31, Nr. 45, 1990, Seiten 6527 - 6530 M.B. GIUDICELLI ET AL

## Beschreibung

Die vorliegende Erfindung betrifft ein neues Verfahren zur Herstellung des bekannten 1-Fluorcyclopropyl-methyl-ketons, das als Zwischenprodukt zur Synthese von Wirkstoffen mit fungiziden Eigenschaften verwendet werden kann.

Es ist bereits bekannt geworden, daß sich 1-Fluorcyclopropyl-methyl-keton herstellen läßt, indem man 2-Acetyl-2-chlor-4-butanolid mit Kaliumfluorid behandelt, das dabei entstehende 2-Acetyl-1-fluor-4-butanolid mit Bromwasserstoff umsetzt und das sich dabei bildende 5-Chlor-3-fluor-pentan-2-on mit Kaliumfluorid cyclisiert (vergl. Synthesis 1977, 189-191). Diese Synthese kann durch das folgende Formelschema veranschaulicht werden: 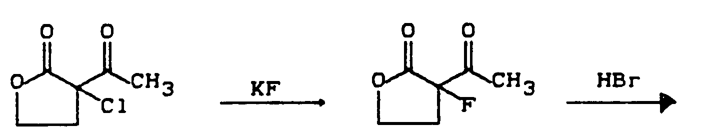 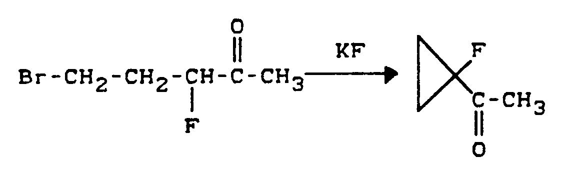
Nachteilig an dem bekannten Verfahren ist, daß es sich um eine dreistufige Synthese handelt und die Ausbeuten bei den einzelnen Reaktionsschritten nur relativ gering sind.

Es wurde nun gefunden, daß man 1-Fluorcyclopropyl-methyl-keton der Formel 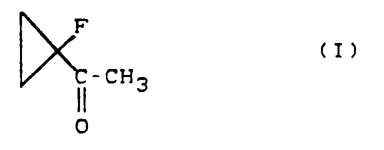
erhält, wenn man
a) in einer ersten Stufe 2-Acetyl-2-chlor-4-butanolid der Formel 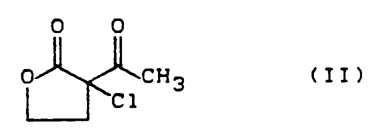 mit einem Triethylamin-Fluorwasserstoff-Additionsprodukt der Formel

   (C₂H₅)₃N . n HF (III)

   in welcher
   - n: für die Zahlen 1, 2 oder 3 steht,
   in Gegenwart eines Verdünnungsmittels bei Temperaturen zwischen 20°C und 120°C umsetzt und
b) in einer zweiten Stufe das entstandene 2-Acetyl-2-fluor-4-butanolid der Formel 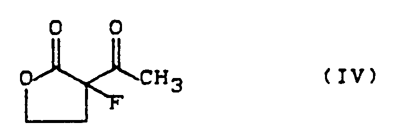 mit einem nucleophilen Agens in Gegenwart eines Verdünnungsmittels bei Temperaturen zwischen 50°C und 200°C umsetzt.

Es ist als äußerst überraschend anzusehen, daß sich 1-Fluorcyclopropyl-methyl-keton der Formel (I) nach dem erfindungsgemäßen Verfahren in glatter Reaktion mit hoher Ausbeute herstellen läßt. Aufgrund des bekannten Standes der Technik war nämlich damit zu rechnen, daß in der ersten Stufe neben einem Austausch des Chloratoms gegen ein Fluoratom am tertiären Kohlenstoffatom auch in beträchtlichem Ausmaß Eliminierungsreaktionen ablaufen würden (vergl. Tetrahedron Letters 31, 6527-6530 (1990)). Unerwartet ist auch der problemlose Verlauf der zweiten Stufe, denn analoge Umsetzungen von Stoffen mit einem Halogenatom an einem tertiären Kohlenstoffatom waren bisher noch nicht bekannt.

Das erfindungsgemäße Verfahren zeichnet sich durch eine Reihe von Vorteilen aus. So ermöglicht es die Herstellung des 1-Fluorcyclopropyl-methyl-ketons in sehr guten Ausbeuten und hoher Reinheit nach einer Methode, die eine Stufe weniger umfaßt als die bisher bekannte Synthese. Ferner sind auch die Ausgangssubstanz und die Reaktionskomponenten in einfacher Weise und auch in größerer Menge zugänglich.

Setzt man 2-Acetyl-2-chlor-4-butanolid in der ersten Stufe mit dem Additionsprodukt aus 1 Mol Triethylamin und 2 Mol Fluorwasserstoff um und verwendet man in der zweiten Stufe Kaliumiodid als nucleophiles Agens, so kann der Verlauf des erfindungsgemäßen Verfahrens durch das folgende Formelschema veranschaulicht werden: 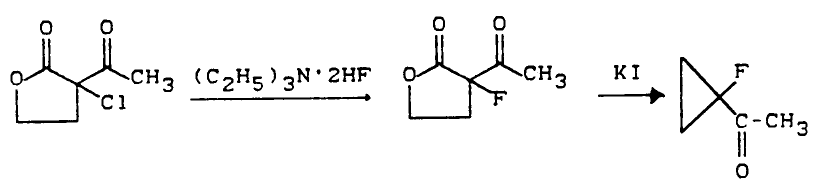
Das bei der Durchführung des erfindungsgemäßen Verfahrens als Ausgangsstoff benötigte 2-Acetyl-2-chlor-4-butanolid der Formel (II) ist bekannt (vergl. Synthesis 1977, 189).

Das bei der Durchführung des erfindungsgemäßen Verfahrens als Reaktionskomponente benötigte Triethylamin-Fluorwasserstoff-Additionsprodukt ist durch die Formel (III) definiert. In dieser Formel steht n für die Zahlen 1, 2 oder 3. Vorzugsweise steht n für die Zahl 2.

Das bei der Durchführung des erfindungsgemäßen Verfahrens in der ersten Stufe als Reaktionskomponente benötigte Triethylamin-Fluorwasserstoff-Additionsprodukt der Formel (III) ist ebenfalls bekannt (vergl. Tetrahedron Letters 31, 6527-6530 (1990)). Es wird im allgemeinen in frisch zubereitetem Zustand verwendet. Man stellt es her, indem man zu einer Lösung des Additionsproduktes aus 1 Mol Triethylamin und 3 Mol Fluorwasserstoff in einem Verdünnungsmittel die berechnete Menge an Triethylamin hinzugibt.

Als nucleophile Reagenzien kommen bei der Durchführung der zweiten Stufe des erfindungsgemäßen Verfahrens alle für derartige Umsetzungen üblichen Substanzen in Betracht. Vorzugsweise verwendbar sind Alkalimetallhalogenide, wie Kaliumiodid, Kaliumbromid, Natriumiodid, Natriumbromid und Natriumchlorid, ferner cyclische Amine, wie 1,4-Diaza-bicyclo[2,2,2]octan (DABCO), und auch Phasentransfern-Katalysatoren, wie Tetramethylammonium-bromid und Triethyl-benzylammonium-chlorid.

Als Verdünnungsmittel kommen bei der Durchführung der ersten Stufe des erfindungsgemäßen Verfahrens alle für derartige Umsetzungen üblichen organischen Solventien in Frage. Vorzugsweise verwendbar sind aliphatische und aromatische, gegebenenfalls halogenierte Kohlenwasserstoffe, wie Hexan, Benzol, Toluol und Chlorbenzol, ferner Ether, wie Tetrahydrofuran und Dioxan, und weiterhin polare Lösungsmittel, wie Acetonitril, Dimethylformamid und N-Methyl-pyrrolidon.

Bei der Durchführung der zweiten Stufe des erfindungsgemäßen Verfahrens kommen als Verdünnungsmittel wiederum alle für derartige Umsetzungen üblichen inerten organischen Solventien in Betracht. Vorzugsweise verwendbar sind hochsiedende organische Lösungsmittel, wie N-Methyl-pyrrolidon, Dimethylsulfoxid, Dimethylformamid, Xylole Hexamethyl-phosphorsäuretriamid, 1,3-Dimethyl-imidazolidin-2-on und andere.

Die Reaktionstemperaturen können bei der Durchführung der ersten Stufe des erfindungsgemäßen Verfahrens innerhalb eines bestimmten Bereiches variiert werden. Im allgemeinen arbeitet man bei Temperaturen zwischen 20°C und 120°C, vorzugsweise zwischen 40°C und 100°C.

Bei der Durchführung der ersten Stufe des erfindungsgemäßen Verfahrens arbeitet man im allgemeinen unter Normaldruck. Es ist aber auch möglich, unter erhöhtem oder vermindertem Druck zu arbeiten.

Bei der Durchführung der ersten Stufe des erfindungsgemäßen Verfahrens setzt man auf 1 Mol an 2-Acetyl-2-chlor-4-butanolid der Formel (II) im allgemeinen 1 bis 5 Mol, vorzugsweise 2 bis 4 Mol an Triethylamin-Fluorwasserstoff-Additionsprodukt der Formel (III) ein. Die Aufarbeitung erfolgt nach üblichen Methoden. Im allgemeinen geht man so vor, daß man das Reaktionsgemisch nach vorheriger Abdestillation eines Teiles des Lösungsmittels mit Wasser versetzt, dann mit einem mit Wasser wenig mischbaren organischen Lösungsmittel extrahiert, die vereinigten organischen Phasen wäscht, trocknet und unter vermindertem Druck destilliert.

Die Reaktionstemperaturen können auch bei der Durchführung der zweiten Stufe des erfindungsgemäßen Verfahrens innerhalb eines bestimmten Bereiches variiert werden. Im allgemeinen arbeitet man bei Temperaturen zwischen 50°C und 200°C, vorzugsweise zwischen 100°C und 180°C.

Bei der Durchführung der zweiten Stufe des erfindungsgemäßen Verfahrens arbeitet man entweder unter Normaldruck oder unter vermindertem Druck.

Bei der Durchführung der zweiten Stufe des erfindungsgemäßen Verfahrens setzt man auf 1 Mol an 2-Acetyl-2-fluor-4-butanolid der Formel (IV) im allgemeinen 0,1 bis 1 Mol, vorzugsweise 0,2 bis 0,8 Mol an nucleophilem Agens ein. Zweckmäßigerweise geht man so vor, daß man 2-Acetyl-2-fluor-4-butanolid bei erhöhter Temperatur und unter vermindertem Druck in eine Lösung eines nucleophilen Agens in einem organischen Solvens eintropft und das entstehende 1-Fluorcyclopropyl-methyl-keton kontinuierlich abdestilliert. Zur weiteren Reinigung kann das Produkt einer erneuten Destillation unterworfen werden.

Das 1-Fluorcyclopropyl-methyl-keton der Formel (I) ist ein wertvolles Zwischenprodukt zur Synthese von Wirkstoffen mit fungiziden Eigenschaften (vergl. EP-OS 0 436 348 und EP-OS 0 297 345). So läßt sich zum Beispiel die Verbindung der Formel 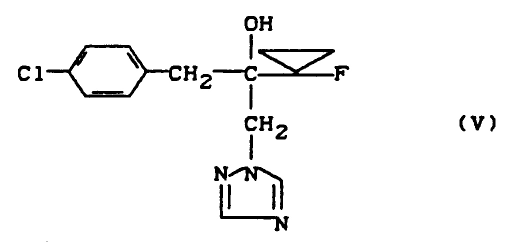
herstellen, indem man 1-Fluorcyclopropyl-methyl-keton der Formel 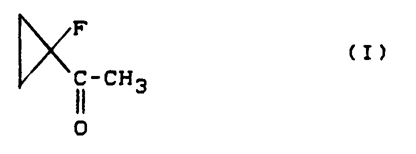
mit Chlorierungs- oder Bromierungsmitteln in Gegenwart eines Verdünnungsmittels umsetzt, die entstehenden Halogenketone der Formel 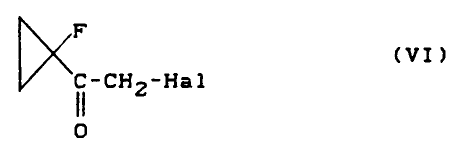
in welcher
- Hal: für Chlor oder Brom steht,
mit 4-Chlorbenzyl-magnesium-chlorid der Formel 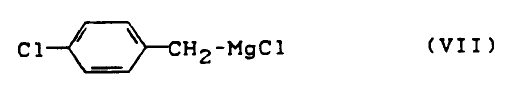
in Gegenwart eines Verdünnungsmittels umsetzt und die dabei anfallenden Propanol-Derivate der Formel 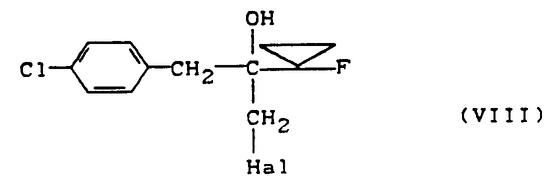
in welcher
- Hal: die oben angegebene Bedeutung hat,
mit 1,2,4-Triazol in Gegenwart eines Verdünnungsmittel und gegebenenfalls in Gegenwart eines Säurebindemittels umsetzt.

Die Durchführung des erfindungsgemäßen Verfahrens wird durch die folgenden Beispiele veranschaulicht.

### Herstellungsbeispiel

### Beispiel 1

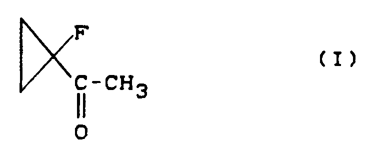

a) Herstellung von 2-Acetyl-2-fluor-4-butanolid der Formel 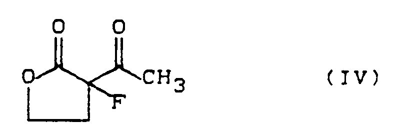 162,6 g (1 Mol) 2-Acetyl-2-chlor-4-butanolid werden in 500 ml Acetonitril gelöst und unter Feuchtigkeitsausschluß mit 340 g (2 Mol) eines Additionsproduktes aus 1 Mol Triethylamin und 3 Mol Fluorwasserstoff versetzt. Danach gibt man 100 g (1 Mol) Triethylamin hinzu und erhitzt das Reaktionsgemisch 3 Stunden unter Rühren auf 80°C. Anschließend destilliert man etwa 400 ml Lösungsmittel ab und gießt den Rückstand auf Wasser. Das entstehende Gemisch wird mit Dichlormethan extrahiert. Die vereinigten organischen Phasen werden mit gesättigter, wäßriger Natriumhydrogencarbonat-Lösung gewaschen, über Magnesiumsulfat getrocknet und unter vermindertem Druck eingeengt. Durch fraktionierte Destillation des verbleibenden Rückstandes unter vermindertem Druck erhält man 95 g (65 % der Theorie) an 2-Acetyl-2-fluor-4-butanolid in Form eines farblosen Öles, das unter einem Druck von 0,15 mbar bei 65°C siedet.
b) Herstellung von 1-Fluorcyclopropyl-methyl-keton der Formel 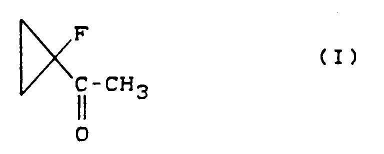 In ein Gemisch aus 16 g (0,1 Mol) Kaliumiodid in 80 ml absolutem N-Methylpyrrolidon werden bei 180°C unter einem Druck von 500 mbar 36,5 g (0,25 Mol) 2-Acetyl-2-fluor-4-butanolid eingetropft. Durch kontinuierliche Abdestillation erhält man 14 g eines Produktes, das gemäß Gaschromatogramm noch zu etwa 20 % aus N-Methyl-pyrrolidon besteht. Das Produkt wird einer fraktionierten Destillation unter Normaldruck unterworfen. Auf diese Weise erhält man 11,2 g (44 % der Theorie) an 1-Fluorcyclopropyl-methyl-keton vom Siedepunkt 88 bis 90°C.

### Verwendungsbeispiel

### Herstellung der Verbindung der Formel

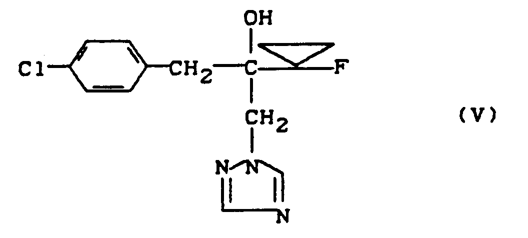

a) Herstellung von 1-Fluorcyclopropyl-chlormethyl-keton der Formel 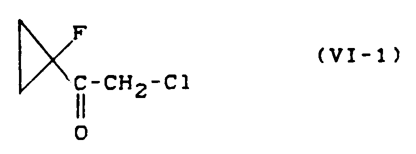 Unter Feuchtigkeitsausschluß werden 67,5 g (0,5 Mol) Sulfurylchlorid bei Raumtemperatur unter Rühren in ein Gemisch aus 40 g (0,4 Mol) 1-Fluorcyclopropyl-methyl-keton und 100 ml Methylenchlorid eingetropft. Nach beendeter Zugabe wird 6 Stunden bei Raumtemperatur nachgerührt. Anschließend gießt man das Reaktionsgemisch auf Eiswasser, trennt die Phasen, wäscht die organische Phase mit Wasser, trocknet über Calciumchlorid und zieht das Lösungsmittel unter vermindertem Druck ab. Der verbleibende Rückstand wird unter vermindertem Druck destilliert. Man erhält auf diese Weise 31,8 g (58 % der Theorie) an 1-Fluorcyclopropyl-chlormethyl-keton in Form eines farblosen Öles.
   Kp = 50°C / 22 mbar
   ¹H-NMR (200 MHz, CDCl₃, TMS-intern.)
   δ = 1,46 ppm (s, 2H); 1,53 ppm (m,2H), 4,65 ppm (d, 2H).
   ¹⁹F-NMR (200 MHz, CDCl₃, CFCl₃)
   δ = 201,7 ppm (m).
b) Herstellung von 1-Chlor-2-(1-fluorcyclopropyl)-3-(4-chlorphenyl)-propan-2-ol der Formel 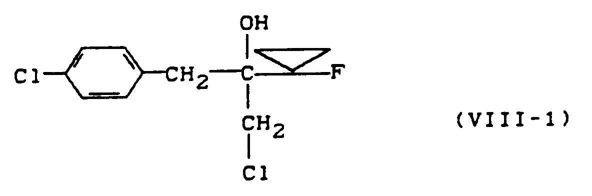 Eine Lösung von 37,3 g (0,25 Mol) 4-Chlor-benzyl-chlorid in 400 ml absolutem Diethylether wird bei Raumtemperatur in ein Gemisch aus 6,8 g (0,28 Mol) Magnesium-Spänen und 150 ml absolutem Diethylether getropft. Man erhitzt 30 Minuten unter Rückfluß und tropft die entstandene Lösung dann bei -78°C unter Rühren in eine Losung von 34,1 g (0,25 Mol) 1-Fluorcyclopropyl-chlormethylketon in 250 ml absolutem Diethylether ein. Das Reaktionsgemisch wird 4 Stunden bei -78°C gerührt. Danach läßt man langsam auf 0°C erwärmen und tropft dann eine Lösung von 25 ml Essigsäure in 250 ml Diethylether hinzu. Das entstandene Reaktionsgemisch wird auf 1000 ml Wasser gegossen. Die organische Phase wird abgetrennt, mit wäßriger Natriumhydrogensulfit-Lösung und mit Wasser gewaschen, über Natriumsulfat getrocknet und unter vermindertem Druck eingeengt. Man erhält auf diese Weise 48,7 g (74 % der Theorie) an 1-Chlor-2-(1-fluorcyclopropyl)-3-(4-chlorphenyl)-propan-2-ol in Form eines öligen Produktes.
c) Herstellung von 1-(4-Chlorphenyl)-2-(1-fluorcylcopropyl)-3-(1,2,4-triazol-1-yl)-propan-2-ol der Formel 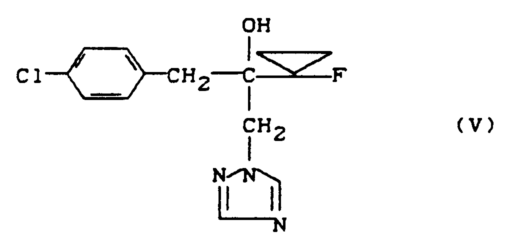 Unter Stickstoffatmosphäre werden 27,6 g (0,4 Mol) 1,2,4-Triazol und 33,6 g (0,3 Mol) Kalium-tert.-butylat in 100 ml absolutem Dimethylformamid vorgelegt und auf 80°C erwärmt. Bei dieser Temperatur tropft man unter Rühren eine Lösung von 26,3 g (0,1 Mol) 1-Chlor-2-(1-fluor-cyclopropyl)-3-(4-chlorphenyl)-propan-2-ol in 50 ml absolutem Dimethylformamid hinzu. Es wird 6 Stunden bei 100°C nachgeführt und dann durch Abziehen des Verdünnungsmittel unter vermindertem Druck eingeengt. Man nimmt den Rückstand in Essigester auf, wäscht mit Wasser und zieht nach dem Trocknen über Natriumsulfat das Lösungsmittel unter vermindertem Druck ab. Das verbleibende Produkt wird über eine Kieselgel-Säule mit Chloroform als Laufmittel chromatographiert. Man erhält auf diese Weise 8,9 g (35 % der Theorie) an 1-(4-Chlorphenyl)-2-(1-fluor-cyclopropyl)-3-(1,2,4-triazol-1-yl)-propan-2-ol in Form einer Festsubstanz.

### Vergleichsbeispiel

### Herstellung von 1-Fluorcyclopropyl-methyl-keton nach dem Verfahren gemäß Synthesis 1977, 189-191:

a) Herstellung von 2-Acetyl-2-fluor-4-butanolid der Formel 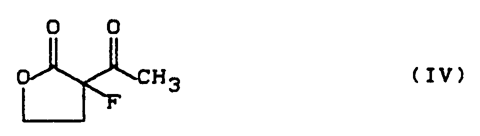 Eine Lösung von 162,5 g (1 Mol) 2-Acetyl-2-chlor-4-butanolid in 700 ml Acetonitril wird mit 174,3 g (3 Mol) Kaliumfluorid und 0,1 g 18-Krone-6 versetzt. Das Reaktionsgemisch wird zunächst 104 Stunden unter Rückfluß erhitzt und dann durch Abdestillieren eines großen Teiles des Lösungsmittels unter Normaldruck eingeengt. Der verbleibende Rückstand wird unter vermindertem Druck bis zur Trockne destilliert. Das aufgefangene Destillat wird noch einmal unter vermindertem Druck über eine 80 cm lange Vigreux-Kolonne destilliert. Man erhält auf diese Weise 37,4 g (26 % der Theorie) an 2-Acetyl-2-fluor-4-butanolid.
b) Herstellung von 5-Brom-3-fluor-pentan-2-on der Formel 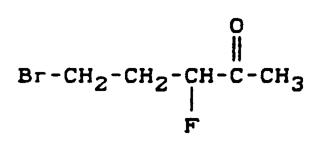 73 g (0,5 Mol) 2-Acetyl-2-fluor-4-butanolid werden mit 280 ml 48 %iger Bromwasserstoffsäure versetzt und 3 Stunden bei 60°C gerührt. Danach gießt man das Gemisch in 400 ml Wasser und extrahiert zweimal mit je 200 ml Diethylether. Die vereinigten organischen Phasen werden nacheinander mit gesättigter, wäßriger Natriumhydrogencarbonat-Lösung und mit gesättigter, wäßriger Natriumchlorid-Lösung gewaschen. Nach dem Trocknen über Molekularsieb wird die organische Phase durch Abziehen des Lösungsmittels unter vermindertem Druck eingeengt. Der verbleibende Rückstand wird unter vermindertem Druck destilliert. Man erhält auf diese Weise 45,6 g (50 % der Theorie) an 5-Brom-3-fluor-pentan-2-on.
c) Herstellung von 1-Fluorcyclopropyl-methyl-keton der Formel 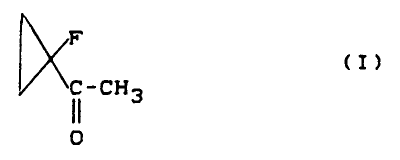 in einem Zweihalskolben mit Magnetrührer, Tropftrichter und Kurzwegdestillationsaufsatz werden 11,6 g (0,2 Mol) Kaliumfluorid und 40 ml Diethylenglykol vorgelegt. Dann werden bei 110°C und 90 mbar 18,3 g (0,1 Mol) 5-Brom-3-fluor-2-pentanon zugetropft, so daß das Produkt laufend abdestilliert. Nach 30 Minuten wird der Druck für 15 Minuten auf 15 mbar reduziert. Das Destillat wird über Magnesiumsulfat getrocknet und über eine Spaltrohr-Kolonne destilliert. Man erhält auf diese Weise 4,1 g (40 % der Theorie) an 1-Fluorcyclopropyl-methyl-keton.

## Patentansprüche

1. Verfahren zur Herstellung von 1-Fluorcyclopropyl-methyl-keton der Formel 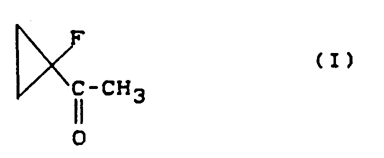 daduch gekennzeichnet, daß man
a) in einer ersten Stufe 2-Acetyl-2-chlor-4-butanolid der Formel 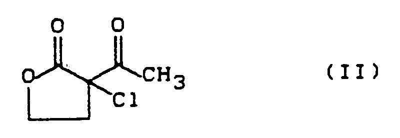 mit einem Triethylamin-Fluorwasserstoff-Additionsprodukt der Formel
(C₂H₅)₃N · n HF (III)
in welcher
n für die Zahlen 1, 2 oder 3 steht,
in Gegenwart eines Verdünnungsmittels bei Temperaturen zwischen 20°C und 12o°C umsetzt und
b) in einer zweiten Stufe das entstandene 2-Acetyl-2-fluor-4-butanolid der Formel 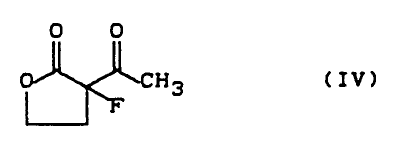 mit einem nucleophilen Agens in Gegenwart eines Verdünnungsmittels bei Temperaturen zwischen 50°C und 200°C umsetzt.

2. Verfahren gemäß Anspruch 1, dadurch gekennzeichnet, daß man bei der Durchführung der zweiten Stufe Alkalimetallhalogenide, cyclische Amine, Tetramethyl-ammonium-bromid oder Triethyl-benzyl-ammonium-chlorid als nucleophile Reagenzien einsetzt.

3. Verfahren gemäß Anspruch 1, dadurch gekennzeichnet, daß man bei der Durchführung der ersten Stufe gegebenenfalls halogenierte aliphatische oder aromatische Kohlenwasserstoffe, Ether oder polare Solventien als Verdünnungsmittel einsetzt.

4. Verfahren gemäß Anspruch 1, dadurch gekennzeichnet, daß man bei der Durchführung der zweiten Stufe hochsiedende organische Solventien als Verdünnungsmittel einsetzt.

5. Verfahren gemäß Anspruch 1, dadurch gekennzeichnet, daß man bei der Durchführung der ersten Stufe bei Temperaturen zwischen 40°C und 100°C und bei der Durchführung der zweiten Stufe bei Temperaturen zwischen 100°C und 180°C arbeitet.

6. Verfahren gemäß Anspruch 1, dadurch gekennzeichnet, daß man bei der Durchführung der ersten Stufe auf 1 Mol an 2-Acetyl-2-chlor-4-butanolid der Formel (II) 1 bis 5 Mol an Triethylamin-Fluorwasserstoff-Additionsprodukt der Formel (III) einsetzt.

7. Verfahren gemäß Anspruch 1, dadurch gekennzeichnet, daß man bei der Durchführung der zweiten Stufe auf 1 Mol an 2-Acetyl-2-fluor-4-butanolid der Formel (IV) 0,1 bis 1 Mol an nucleophilem Agens einsetzt.

8. Verfahren gemäß Anspruch 1, dadurch gekennzeichnet, daß als Reaktionskomponente der Formel (III) ein Additionsprodukt aus 1 Mol Triethylamin und 2 Mol Fluorwasserstoff eingesetzt wird.

## Claims

1. Method for preparing 1-fluorocyclopropyl methyl ketone, of the formula 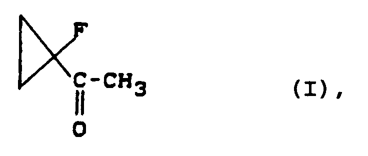 characterised in that
a) in a first step, 2-acetyl-2-chloro-4-butanolide, of the formula 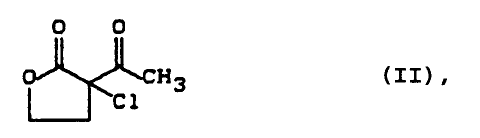 is reacted with an addition product of triethylamine and hydrogen fluoride of the formula
(C₂H₅)₃N· n HF (III)
in which
n represents the numbers 1, 2 or 3,
in the presence of a diluent at temperatures between 20°C and 120°C and
b) in a second step, the 2-acetyl-2-fluoro-4-butanolide, of the formula 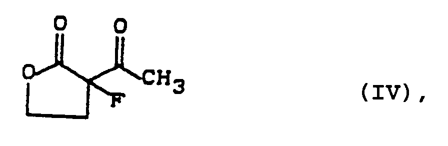 produced is reacted with a nucleophilic agent in the presence of a diluent at temperatures between 50°C and 200°C.

2. Method according to Claim 1, characterised in that, in carrying out the second step, alkali metal halides, cyclic amines, tetramethylammonium bromide or triethyl-benzyl-ammonium chloride are used as nucleophilic reagents.

3. Method according to Claim 1, characterised in that, in carrying out the first step, optionally halogenated aliphatic or aromatic hydrocarbons, ethers or polar solvents are used as diluents.

4. Method according to Claim 1, characterised in that, in carrying out the second step, high-boiling organic solvents are used as diluents.

5. Method according to Claim 1, characterised in that, in carrying out the first step, temperatures between 40°C and 100°C, and, in carrying out the second step, temperatures between 100°C and 180°C are used.

6. Method according to Claim 1, characterised in that, in carrying out the first step, 1 to 5 mol of the addition product of the formula (III) of triethylamine and hydrogen fluoride are used per 1 mol of 2-acetyl-2-chloro-4-butanolide of the formula (II).

7. Method according to Claim 1, characterised in that, in carrying out the second step, 0.1 to 1 mol of nucleophilic agent is used per 1 mol of 2-acetyl-2-fluoro-4-butanolide of the formula (IV).

8. Method according to Claim 1, characterised in that an addition product of 1 mol of triethylamine and 2 mol of hydrogen fluoride is used as the reactant of the formula (III).

## Revendications

1. Procédé pour la préparation de la 1-fluorocyclopropylméthylcétone de formule 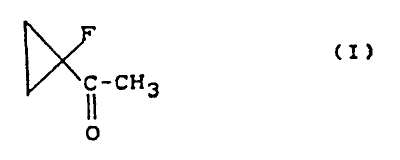 caractérisé en ce que
a) dans une première étape, on fait réagir le 2-acétyl-2-chloro-4-butanolide de formule 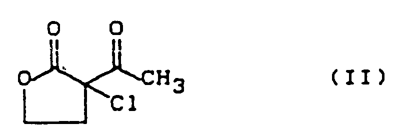 avec un produit d'addition de triéthylamine-acide fluorhydrique répondant à la formule
(C₂H₅)₃N . n HF (III)
dans laquelle
n représente les nombres 1, 2 ou 3,
en présence d'un diluant, à des températures entre 20°C et 120°C, et
b) dans une seconde étape, on fait réagir le 2-acétyl-2-fluoro-4-butanolide obtenu de formule 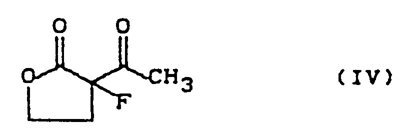 avec un agent nucléophile en présence d'un diluant, à des températures entre 50°C et 200°C.

2. Procédé selon la revendication 1, caractérisé en ce que, lors de la mise en oeuvre de la seconde étape, on met en oeuvre des halogénures de métaux alcalins, des amines cycliques, du bromure de tétraméthylammonium ou du chlorure de triéthylbenzylammonium comme réactifs nucléophiles.

3. Procédé selon la revendication 1, caractérisé en ce que, lors de la mise en oeuvre de la première étape, on met en oeuvre des hydrocarbures aliphatiques ou aromatiques éventuellement halogénés, des éthers ou encore des solvants polaires comme diluant.

4. Procédé selon la revendication 1, caractérisé en ce que, lors de la mise en oeuvre de la seconde étape, on met en oeuvre des solvants organiques à point d'ébullition élevé, comme diluant.

5. Procédé selon la revendication 1, caractérisé en ce que, dans la mise en oeuvre de la première étape, on travaille à des températures entre 40°C et 100°C, et lors de la mise en oeuvre de la seconde étape, on travaille à des températures entre 100°C et 180°C.

6. Procédé selon la revendication 1, caractérisé en ce que, lors de la mise en oeuvre de la première étape, pour 1 mole du 2-acétyl-2-chloro-4-butanolide de formule (II), on met en oeuvre de 1 à 5 moles de produit d'addition de triéthylamine-acide fluorhydrique de formule (III).

7. Procédé selon la revendication 1, caractérisé en ce que, lors de la mise en oeuvre de la seconde étape, pour 1 mole du 2-acétyl-2-fluoro-4-butanolide de formule (IV), on met en oeuvre de 0,1 à 1 mole d'agent nucléophile.

8. Procédé selon la revendication 1, caractérisé en ce que, comme composant réactionnel de formule (III), on met en oeuvre un produit d'addition de 1 mole de triéthylamine et de 2 moles d'acide fluorhydrique.
